# EUROPEAN PATENT SPECIFICATION

(11) **EP 3 125 764 B1**
(45) Date of publication and mention of the grant of the patent: **22.03.2023**
(21) Application number: 15712112.0
(22) Date of filing: 23.03.2015
(51) Int. Cl.: A61B 6/03, A61B 5/02

(54) **PROCESSING APPARATUS FOR PROCESSING CARDIAC DATA OF A LIVING BEING**
VERARBEITUNGSVORRICHTUNG ZUR VERARBEITUNG VON HERZDATEN EINES LEBEWESENS
APPAREIL DE TRAITEMENT DE DONNÉES CARDIAQUES D'UN ÊTRE VIVANT

(30) Priority: 31.03.2014 EP 14162702
(43) Date of publication of application: 08.02.2017
(73) Proprietor: Koninklijke Philips N.V., 5656 AG Eindhoven (NL)
(72) Inventor: SCHMITT, Holger, NL-5656 AE Eindhoven (NL); PREVRHAL, Sven, NL-5656 AE Eindhoven (NL); NICKISCH, Hannes, NL-5656 AE Eindhoven (NL)
(74) Representative: Philips Intellectual Property & Standards
(86) International application number: PCT/EP2015/056036
(87) International publication number: WO 2015/150128

(56) References cited:
- US-A1- 2012 053 918
- US-A1- 2013 324 842

## Description

### FIELD OF THE INVENTION

The present invention relates to a processing apparatus for processing cardiac data of a living being and an apparatus for evaluating cardiac data of a living being.

### BACKGROUND OF THE INVENTION

Degenerative stenosis is the second most common cardiovascular disease with an incidence of 2-7% in the Western European and North American populations aged beyond 65 years, as described in G.M. Feuchtner, W. Dichtl, et al. "Multislice Computed Tomography for Detection of Patients With Aortic Valve Stenosis and Quantification of Severity", Journal of the American College of Cardiology 2006, 47 (7), 1410-1417. In the context of the present invention the term stenosis represents any abnormal narrowing of an artery. In interventional cardiology a degree of stenosis may be measured using fractional flow reserve (FFR) techniques in which a catheter is introduced into a coronary artery, which is able to measure a relative difference between pressure behind (distal to) and before (proximal to) a stenosis in the artery. The higher the pressure drop, the more severe the stenosis is. A distal/proximal pressure ratio of FFR<0.8 is usually considered as flow limiting.

Simulation methods have become available in which the FFR value is calculated on the basis of non-invasive medical imaging (such as computed tomography, NMR, PET and the like) to determine a degree of stenosis by performing FFR calculations based on reconstructed arterial information, such as is known from, for instance, US 2012/053918 A1. This 'virtual FFR value' may then be used as input for a cardiologist to determine a further course of action (e.g. prescribe medication or invasive treatment, such as surgery or stenting). In case follow-up action requires catheterization, e.g. for further diagnosis, actual FFR values may be measured form in-artery measurements.

Regularly it is found that the calculated FFR values differ from the measured FFR values, e.g. due to imaging errors, such as artifacts, or limitations in reconstruction algorithms. In some cases the virtual FFR values might differ significantly from the measured FFR values, such that it warrants a different course of action for the cardiologist, such as deciding on a different treatment or exploring different areas of the patient's arterial system. This may cause a significant increase in time needed for diagnosis or treatment. It would therefore be desirable if this increase can be avoided, or at least reduced.

US 2012/0053918 A1 discloses a system for planning a treatment for a patient, wherein the system includes a computer system configured to receive patient-specific data regarding a geometry of an anatomical structure of the patient, create a three-dimensional model representing at least a portion of the anatomical structure of the patient based on the patient-specific data, and determine a first fractional flow reserve within the anatomical structure of the patient based on the three-dimensional model and a physics-based model relating to the anatomical structure of the patient.

### SUMMARY OF THE INVENTION

It is an object of the present invention to provide a processing apparatus for processing cardiac data of a living being which provides more reliable FFR values that will reduce the need to change the course of action of a cardiologist and will result in a reduced diagnosis or treatment time.

In a first aspect of the present invention a processing apparatus for processing cardiac data of a living being according to claim 1 is presented.

The processing apparatus facilitates checking of a simulated FFR value against an actual measured FFR for the same position in the living being's arterial system. The correction unit corrects the simulated FFR value for a certain position in the arterial system of the living being using a measured FFR value for that same position. This has several advantages. Most importantly, the speed and reliability of diagnosis and flexibility of treatment by a cardiologist is improved. Furthermore, the quality of the calculation model is checked by comparing the calculated values with actual measured values, which may result in improved future models.

In a preferred embodiment of the processing apparatus of the present invention the correction unit corrects a simulated FFR value for a location in the arterial system by replacing this value with a measured FFR value that was measured on the same location. This correction is simple to implement and provides the cardiologist and the calculation model with actual measured data instead of calculated data, which reflects the actual situation more realistically than the calculated FFR values.

In a further preferred embodiment of the processing apparatus of the present invention fractional flow values are (re-)calculated at different positions in the arterial system than the position of the measured fractional flow value. These (re-)calculated FFR values are based on improved input data and therefore more likely to be closer to actual values. This increases the reliability and speed and thereby further assists the cardiologist in his diagnosis or treatment. The accuracy, and therefore the reliability, of the calculation model and the simulated FFR values will drastically improve with each new measurement.

The processing apparatus according to the present invention further comprises an arterial tree model providing unit. An arterial tree model of the living being is constructed from non-invasive imaging data of the coronary arteries (e.g. a CT scan, such as the CT scan that provided the simulated FFR values), from data obtained from in-artery imaging (e.g. from a camera mounted on a catheter, such as the catheter with which the measured FFR values were obtained or from a combination of non-invasive and in-artery imaging. The arterial model may be displayed by the display unit with calculated and or measured FFR values. This provides the cardiologist with realistic graphic information and relevant FFR values in relation to their actual position.

It is preferred that the correction unit automatically corrects simulated flow reserve model values when a new measured FFR value becomes available. This may be replacing the simulated FFR value by the measured value and/or (re-)calculating simulated FFR values at different positions in the arterial tree. It is further preferred that the display unit displays the corrected FFR values as soon as possible after the corrected value becomes available. This assists the cardiologist to adapt his strategy, if necessary, as soon as possible. When the FFR values are calculated within 10 seconds from obtaining the imaging data a more-or-less real-time situation may be presented to the cardiologist. More preferably the values are calculated within 5 seconds, even more preferably within 2 seconds and even more preferably within 1 second. Most preferred would be as close to an instantaneous calculation that is technically possible.

In a further aspect of the present invention an apparatus for evaluating cardiac data of a living being according to claim 5 is presented.

### BRIEF DESCRIPTION OF THE DRAWINGS

In the following drawings:
Fig 1 schematically and exemplarily shows an embodiment of a processing apparatus according to the present invention;
Fig 2 schematically and exemplarily shows a further embodiment of a processing apparatus according to the present invention; and
Fig 3a-f schematically and exemplary show an embodiment of information presented to a cardiologist over time when measured FFR values become available; and
Fig 4 shows a flow chart for an embodiment of a method according to the present disclosure.

### DETAILED DESCRIPTION OF EMBODIMENTS

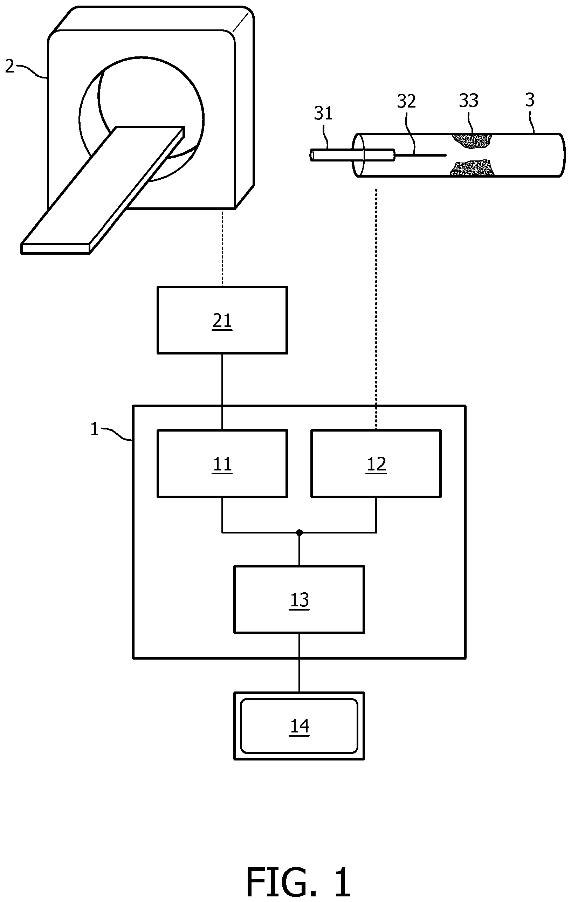

Fig. 1 shows, schematically and exemplarily, an embodiment of a processing apparatus according to the present invention. In this embodiment the processing apparatus 1 comprises a first FFR value providing unit 11, a second FFR value providing unit 12, a correction unit 13. The FFR values are displayed with display unit 14.

The first FFR value providing unit 11 provides simulated FFR values obtained from an image processing unit 21 that processes images obtained from a non-invasive imaging device 2, such as a computed tomography imaging device, an ultrasound imaging device, a positron emission tomography imaging device, a magnetic resonance imaging device, an x-ray imaging device and other non-invasive imaging devices known to the skilled person or combinations thereof. The image processing unit 21 determines first FFR values from detection data detected by the non-invasive imaging device 2, e.g. by simulating the first FFR values through calculations based on reconstructed arterial information, such as coronary artery information, as is known in the art. Preferably, the first FFR value providing unit provides first FFR values for various locations in the arterial tree.

The second FFR value providing unit 12 provides FFR values obtained from in-artery measurements, e.g. measurements performed by a pressure wire 32 that is forwarded through a catheter 31 which is inserted into an artery 3, such as a coronary artery, wherein an arterial pressure is measured at at least two locations in the artery, e.g. before and after a known stenosis 33 in the artery 3. The second FFR value is then determined as the ratio between the pressure measured at the two locations. The location of the stenosis 33 may have been initially determined by visual inspection of reconstructed image data from a non-invasive imaging device or by through image analysis algorithms analyzing said reconstructed image data. Preferably, the reconstructed image data is obtained simultaneously or at least recently before obtaining the first FFR values using the same non-invasive imaging device 2.

In correction unit 13 the provided first and second FFR values for a location in the artery 3 are compared to each other and, if necessary, the first FFR value is corrected. It is reasonably assumed that the second FFR value reflects the actual situation in the artery 3 better than the first FFR value, since the second FFR value is an actual measured value, while the first FFR is only indirectly determined from the image data using algorithms that use imaging data and assumptions that inherently may cause errors in calculating the first FFR value. Therefore, the correction unit 13 gives more weight to the second FFR value, preferably it will simply replace the first FFR value by the second FFR value. This is the most straightforward and easy to implement correction that is possible. However, other corrections may be used as well, such as taking into account the location where the first and second FFR values were determined if they do not exactly align.

In the invention the correction unit corrects a first FFR value at a position for which a second FFR value is not or not yet available by recalculating said first FFR value using at least one second FFR value of a different position in the coronary arteries of the living being. When measured data becomes available for a certain location it does not only improve the first FFR value at that location, it may also serve as a more realistic basis for (re-)calculating FFR values at other positions, e.g. further, in the artery 3, which results in more reliable and accurate information that is available to the cardiologist.

Furthermore, in a preferred embodiment the correction unit 13 only corrects the first FFR value when the difference between the first and second FFR value is above a predetermined threshold value, e.g. the difference is more than 1, 2, 5 or 10%. A difference below this threshold likely does not change the cardiologist's course of action, while time is saved by not needing to correct or recalculate first FFR values.

Display unit 14 displays the first and second FFR values to the cardiologist. This may done in the form of a table, a graphic image or any other suitable presentation of information. For instance, Example 1 shows how the information may be presented in the form of a table.

### EXAMPLE 1

**Table 1:**

| **Coronary Segment** | **Simulated FFR** | **Measured FFR** |
|---|---|---|
| Proximal LAD | 0.6 | ***pending...*** |
| Distal LCX | 0.75 | ***pending...*** |
| Proximal D1 | 0.8 | ***pending...*** |

Table 1 shows an initial situation of first (simulated) and second (measured) FFR values of three different coronary segments (Proximal LAD: Proximal left anterior descending, Distal LCX: Distal left circumflex artery, Proximal D1: Proximal Diagonal 1) that were identified as critical (FFR<= 0.8). In another embodiment also non-critical segments may be shown. In this initial situation first FFR values were determined, but second FFR are not yet available, for instance because the in-artery measurement procedure has not yet begun or the measurement module has not yet reached these segments. The first FFR values are presented, while the phrase 'pending' is presented for the second FFR values. Other phrases, color codes, symbols (e.g. an hourglass) or simply a blank space are possible as well.

**Table 2:**

| **Coronary Segment** | **Simulated FFR** | **Measured FFR** |
|---|---|---|
| Proximal LAD | (0.6) | ***0.55*** |
| Distal LCX | 0.85 | ***pending...*** |
| Proximal D1 | 0.88 | ***pending...*** |

Table 2 shows the situation after an invasive measurement has been made for "Proximal LAD" and the simulation has been re-run. Simulated FFR has changed to 0.85 for "Distal LCX" and 0.88 for "Proximal D1". The cardiologist may decide to skip invasive evaluation of these two segments as the simulation indicates that they are of secondary importance, thereby not extending the procedure unnecesarily. Of course, should the cardiologist decide he may still measure the FFR values at these sites, which then will further improve the reliability and accuracy of the presented information. The measurement confirmed that the "Proximal LAD" segment indeed has a critical FFR value, even lower than the simulated FFR. The correction unit 13 corrects the FFR value such that it will only take into account the measured FFR value for further calculations. The simulated FFR value for "Proximal LAD" is now shown between brackets to alert the cardiologist that this value has been corrected. Alternatively, it may also be replaced by the measured FFR value, blanked out, color coded or it may be indicated in another way that this value is not current anymore.

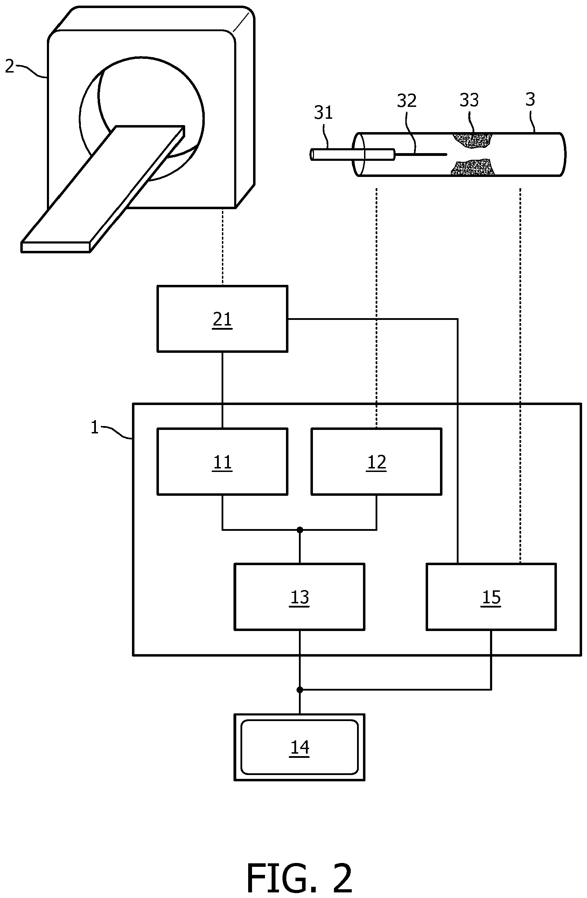

Fig. 2 shows, schematically and exemplarily, a further embodiment of a processing apparatus according to the present invention. This embodiment corresponds to the one shown in Fig. 1, but in addition the processing apparatus comprises an arterial tree providing unit 15. In this embodiment the arterial tree providing unit 15 provides a (3D-)modeled arterial tree of the living being or a segment thereof based on image data reconstructed in image processing unit 21. Said image data also formed the basis for the determination of the first FFR values, thereby ensuring an optimal match between the arterial tree information and the first FFR values, but it is also possible to use previously acquired image data, thereby reducing the examination time and radiation dose. Additionally or alternatively, the image data may (also) be based on imaging data acquired from in artery imaging, e.g. from a catheterized camera, for instance mounted on measuring catheter 31. This will provide an actual image from inside the artery, which may be used to improve the arterial tree model.

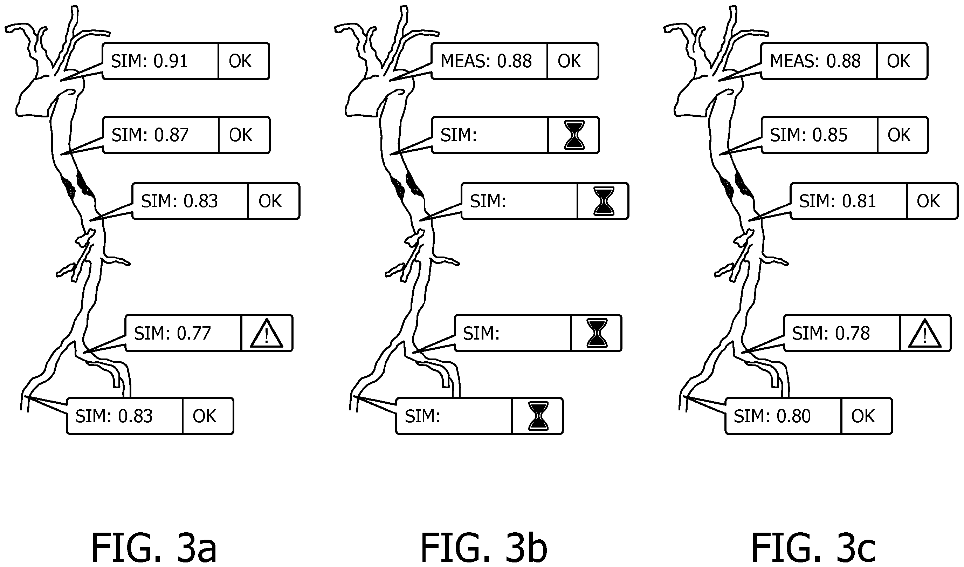

The arterial tree may be displayed by the display unit 14 to assist the cardiologist in determining stenosis locations. In a particularly preferred embodiment the first and second FFR values are displayed at or near the locations in the arterial tree where they were determined or measured. An illustrative example of this is given in Fig. 3a-3f. This example is not meant as a realistic representation of the procedure, but as a simplified way of illustrating the present invention.

In Fig. 3a a 3D model of an arterial tree segment is shown with first (simulated) FFR values for 5 positions in the arterial tree (indicated by the acronym SIM and the FFR value). When an FFR value is higher than a critical value (in this case FFR > 0.8) it is indicated by the word 'OK'. In case an FFR value is critical (in this case FFR <= 0.8) a warning sign is shown. Alternatively this could be indicated differently, e.g. by flashing the critical values or by color coding in two or more colors, such as red for critical FFR, green for non-critical FFR and optionally orange for borderline cases (e.g. 0.8 < FFR 0.85). In Fig. 3a the simulated FFR values show that there is one critical FFR value at the fourth position from the top.

In Fig. 3b a second (measured) FFR value has become available for the top position. In this example the simulated FFR value was replaced by the measured FFR value (indicated by the acronym MEAS and the new FFR value). Alternatively the original simulated FFR value may still be available (similar to the embodiment of Example 1 or other ways of indicating that the FFR value has been corrected for that location in the arterial tree). The FFR values of the other four shown locations are recalculated using the corrected FFR value for the top position. While the recalculation is proceeding, the cardiologist is informed of this by an hourglass symbol. Alternatively, this could be done in different ways (e.g. by using the phrase 'pending', 'recalculating' or the like or by color coding) or not at all (meaning the value is simply replaced when the new value is available).

Fig. 3c shows the result of the recalculated FFR values. Using the corrected, more reliable FFR value as the starting point confirms that the critical FFR value for the fourth position from the top. The recalculated FFR values for the 3^{rd} and 5^{th} positions from the top are still classified as not being critical, but they are so close to the critical value that further investigation will likely be considered by the cardiologist.

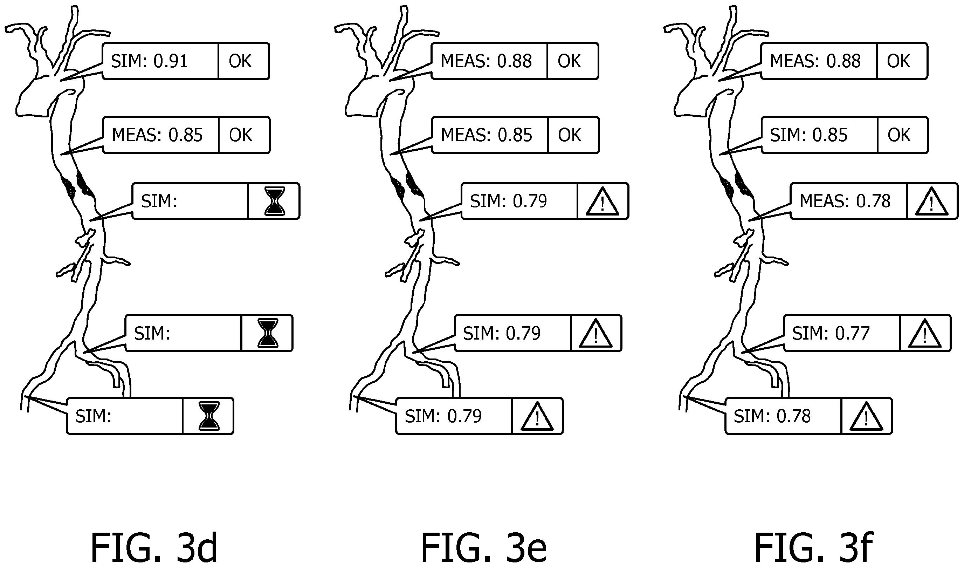

In Fig. 3d also a measured FFR value of the second position has become available, confirming the non-critical value for that position. Again the remaining simulated FFR values are recalculated. The result of which is shown in Fig. 3e, which now shows the corrected FFR values for the 3rd, 4th and 5th position from the top. Using the more accurate starting point for the calculations it is found that now also the 3^{rd} and 5^{th} positions from the top dropped below the critical value of 0.8. Since the simulated FFR value for 5th position from the top has become critical, further examination and measurement for that particular arterial branch is warranted, which might not have been done if only previous, less reliable simulated values would have been available. Should the simulated FFR value for that position be (clearly) non-critical, examination time could be reduced by not having to measure that particular branch.

In Fig. 3f the FFR value for the third position from the top has also been measured and the remaining two simulated FFR values were recalculated. The measured FFR for the third position is closer to the simulated value based on the previous measurement than the initial simulated value, which should be the case since the starting points for the calculations is much more accurate and reliable. This would likely influence treatment choices to avoid possible hazardous consequences of the arterial narrowing.

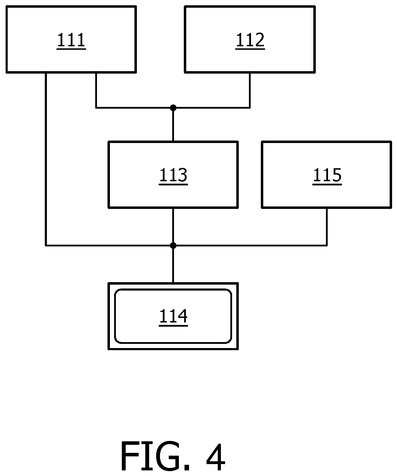

Fig. 4 shows a flow chart for a method for assessment of stenosis in an artery of a living being, comprising the steps of receiving 111 first fractional flow reserve values of the arterial tree calculated from data obtained from non-invasive imaging of the arterial tree; displaying 114 the first fractional flow reserve in relation to the position in the artery; receiving 112 the second fractional flow reserve values of the arterial tree obtained from in-artery measurements; correcting (113) the first fractional flow reserve values based on the second fractional flow reserve values; and displaying 114 the second fractional flow reserve in relation to the position in the artery.

In a preferred embodiment, the method further comprises providing 115 an arterial tree model providing unit and wherein the first and second fractional flow reserve data is graphically displayed 114 in relation to the position in the arterial tree.

In a further preferred embodiment, the method further comprises calculating a first fractional reserve value at a position for which a second fractional flow reserve value is not or not yet available using at least one second fractional flow reserve value of a different position in the coronary arteries of the living being.

The method may be executed by a computer program product comprising instructions to execute the steps of the method when the computer program product is run on the computer.

While the invention has been illustrated and described in detail in the drawings and foregoing description, such illustration and description are to be considered illustrative or exemplary and not restrictive; the invention is limited by the subject-matter of the claims.

In the claims, the word "comprising" does not exclude other elements or steps, and the indefinite article "a" or "an" does not exclude a plurality. A single processor or other unit may fulfill the functions of several items recited in the claims.

The mere fact that certain measures are recited in mutually different dependent claims does not indicate that a combination of these measured cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A processing apparatus for processing cardiac data of a living being, wherein the apparatus comprises
- a first fractional flow reserve value providing unit (11) configured to provide first fractional flow reserve values being indicative of the fractional flow reserve of different arteries (3) of the living being, wherein said first fractional flow reserve values were calculated from non-invasive imaging data of arteries of the living being;
- a second fractional flow reserve value providing unit (12) configured to provide second fractional flow reserve values measured in the arteries (3) of the living being by in-artery measurements; and
- a correction unit configured (13) to correct the provided first fractional flow reserve values based on the provided second fractional flow reserve values, wherein the correction unit (13) is configured to correct a first fractional flow reserve value at a position for which a second fractional flow reserve value is not or not yet available by recalculating said first fractional flow reserve value using at least one second fractional flow reserve value of a different position in the coronary arteries (3) of the living being, and wherein the correction unit (13) is configured to correct the first fractional flow reserve values whenever a new second fractional flow reserve value is provided;
- an arterial tree model providing unit (15) configured to provide an arterial tree model from the non-invasive imaging data of the coronary arteries (3) of the living being and in-artery imaging; and
- a display unit (14) configured to display the arterial tree and to display the first and second fractional flow reserve values at or near the locations in the arterial tree where they were determined or measured.

2. Processing apparatus according to claim 1, wherein the correction unit (13) corrects at least one first fractional flow reserve value for a position in the coronary arteries (3) of the living being by replacing it with a second fractional flow reserve value that is measured at the same position in the coronary arteries (3) of the living being.

3. Processing apparatus according to any of the previous claims, wherein the correction unit (13) only corrects a first fractional flow reserve value when the difference between the first fractional flow reserve value and the second fractional flow reserve value is above a predetermined threshold value.

4. Processing apparatus according to any of the previous claims, wherein first fractional flow reserve values were calculated within 10 seconds from obtaining the non-invasive imaging data of the coronary arteries (3) of the living being.

5. An apparatus for evaluating cardiac data of a living being, comprising
- a processing apparatus (1) according to any of the claims 1 to 4; and
- a display unit (14) configured to display at least one first fractional flow reserve value and a corresponding second fractional flow reserve value for a corresponding position in the coronary arteries (3).

6. An apparatus according to claim 5, further comprising
- an imaging device (2) for non-invasive imaging a cardiac region of a living being; the imaging device (2) comprising a processing unit (21) for determining fractional flow reserve values; and
- a measurement device (31) for in-artery measuring of fractional flow reserve values.

7. An apparatus according to claim 5, wherein the imaging device (2) further comprises an image processing unit (21) for reconstructing three dimensional image data from detection data detected by the imaging device (2).

8. An apparatus according to any of the claims 5 to 7, wherein the display unit (14) is configured to display corrected fractional flow reserve values whenever a first fractional flow reserve value is corrected.

## Patentansprüche

1. Verarbeitungsapparat zur Verarbeitung von Herzdaten eines Lebewesens,
wobei der Apparat Folgendes umfasst
- eine Einheit (11) zur Bereitstellung eines ersten Wertes fraktionaler Durchflussreserve, die so konfiguriert ist, dass sie erste Werte fraktionaler Durchflussreserve bereitstellt, die die Werte fraktionaler Durchflussreserve verschiedener Arterien (3) des Lebewesens anzeigen, wobei die ersten Werte fraktionaler Durchflussreserve aus nicht-invasiven Bildgebungsdaten von Arterien des Lebewesens berechnet wurden;
- eine zweite Einheit (12) zur Bereitstellung von Werten für die fraktionale Durchflussreserve, die so konfiguriert ist, dass sie zweite Werte für die fraktionale
Durchflussreserve bereitstellt, die in den Arterien (3) des Lebewesens durch Messungen in der Arterie gemessen werden; und
- eine Korrektureinheit (13), die so konfiguriert ist, dass sie die bereitgestellten ersten Werte fraktionaler Durchflussreserve auf der Grundlage der bereitgestellten zweiten Werte fraktionaler Durchflussreserve korrigiert, wobei die Korrektureinheit (13) so konfiguriert ist, dass sie einen ersten Wert fraktionaler Durchflussrese an einer Position zu
korrigieren, für die ein zweiter Wert fraktionaler Durchflussreserve nicht oder noch nicht verfügbar ist, indem der erste Wert fraktionaler Durchflussreserve mindestens einen zweiten Wert fraktionaler Durchflussreserve
an einer anderen Position in den Koronararterien (3) des Lebewesens zu verwenden, und wobei die Korrektureinheit (13) so konfiguriert ist, dass sie die ersten Werte fraktionaler Durchflussreserve korrigiert, wenn ein neuer
zweiter Wert fraktionaler Durchflussreservewert bereitgestellt wird;
- eine Einheit (15) zur Bereitstellung eines Arterienbaummodells, die so konfiguriert ist, dass sie ein Arterienbaummodell aus den nicht-invasiven Bildgebungsdaten der Koronararterien (3) des Lebewesens und der Bildgebung in der Arterie erstellt; und
- eine Anzeigeeinheit (14), die so konfiguriert ist, dass sie den Arterienbaum anzeigt und die ersten und zweiten Werte der Durchflussreservewerte an oder in der Nähe der Stellen in dem Arterienbaum anzeigt, an denen sie bestimmt oder gemessen wurden.

2. Verarbeitungsapparat nach Anspruch 1, wobei die Korrektureinheit (13) mindestens einen ersten Wert fraktionaler Durchflussreserve für eine Position in den Koronararterien (3) des Lebewesens korrigiert, indem sie ihn durch einen zweiten Wert fraktionaler Durchflussreserve ersetzt, der an der gleichen Position in den Koronararterien (3) des Lebewesens gemessen wird.

3. Verarbeitungsapparat nach einem der vorhergehenden Ansprüche, wobei die Korrektureinheit (13) einen ersten Wert fraktionaler Durchflussreserve nur dann korrigiert, wenn die Differenz zwischen dem ersten Wert fraktionaler Durchflussreserve und dem zweiten Wert fraktionaler Durchflussreserve über einem vorgegebenen Schwellenwert liegt.

4. Verarbeitungsapparat nach einem der vorhergehenden Ansprüche, wobei erste Werte fraktionaler Durchflussreserve innerhalb von 10 Sekunden nach Erhalt der nicht-invasiven Bildgebungsdaten der Koronararterien (3) des Lebewesens berechnet wurden.

5. Apparat zur Auswertung von Herzdaten eines Lebewesens, der Folgendes umfasst
- ein Bearbeitungsgerät (1) nach einem der Ansprüche 1 bis 4; und
- eine Anzeigeeinheit (14), die so konfiguriert ist, dass sie mindestens einen ersten Wert fraktionaler Durchflussreserve und einen entsprechenden zweiten Wert fraktionaler Durchflussreserve für eine entsprechende Position in den Koronararterien (3) anzeigt.

6. Apparat nach Anspruch 5, der des Weiteren Folgendes umfasst
- ein Bildgebungsgerät (2) zur nicht-invasiven Bildgebung eines Herzbereichs eines Lebewesens; wobei das Bildgebungsgerät (2) eine Verarbeitungseinheit (21) zur Bestimmung von Werten der fraktionalen Durchflussreserve umfasst; und
- ein Messgerät (31) zur arterieninternen Messung der Werte der fraktionalen Durchflussreserve.

7. Apparat nach Anspruch 5, wobei das Bildgebungsgerät (2) ferner eine Bildverarbeitungseinheit (21) zum Rekonstruieren dreidimensionaler Bilddaten aus von der Bildgebungsvorrichtung (2) erfassten Erfassungsdaten umfasst.

8. Apparat nach einem der Ansprüche 5 bis 7, wobei die Anzeigeeinheit
(14) so konfiguriert ist, dass sie korrigierte Bruchteil-Durchflussreservewerte anzeigt,
wenn ein erster Bruchteil-Durchflussreservewert korrigiert wird.

## Revendications

1. Appareil de traitement pour le traitement de données cardiaques d'un être vivant, dans lequel l'appareil comprend :
- une première unité de fourniture de valeurs de réserve de débit fractionnaire (11) configurée pour fournir des premières valeurs de réserve de débit fractionnaire indicatives de la réserve de débit fractionnaire de différentes artères (3) de l'être vivant, dans laquelle lesdites premières valeurs de réserve de débit fractionnaire ont été calculées à partir de données d'imagerie non invasive d'artères de l'être vivant;
- une deuxième unité de fourniture de valeurs de réserve de débit fractionnaire (12) configurée pour fournir des deuxièmes valeurs de réserve de débit fractionnaire mesurées dans les artères (3) de l'être vivant par des mesures dans les artères; et
- une unité de correction configurée (13) pour corriger les premières valeurs de réserve de débit fractionnaire fournies sur la base des deuxièmes valeurs de réserve de débit fractionnaire fournies, dans laquelle l'unité de correction (13) est configurée pour corriger une première valeur de réserve de débit fractionnaire à une position pour laquelle une deuxième valeur de réserve de débit fractionnaire n'est pas ou pas encore disponible en recalculant ladite première valeur de réserve de débit fractionnaire en utilisant au moins une deuxième valeur de réserve de débit fractionnaire d'une position différente dans les artères coronaires (3) de l'être vivant, et dans lequel l'unité de correction (13) est configurée pour corriger les premières valeurs de réserve de débit fractionnaire chaque fois qu'une nouvelle deuxième valeur de réserve de débit fractionnaire est fournie ;
- une unité de fourniture de modèle d'arbre artériel (15) configurée pour fournir un modèle d'arbre artériel à partir des données d'imagerie non invasive des artères coronaires (3) de l'être vivant et de l'imagerie intra-artérielle; et
- une unité d'affichage (14) configurée pour afficher l'arbre artériel et pour afficher les première et deuxième valeurs de réserve de débit fractionnaire à ou près des emplacements dans l'arbre artériel où elles ont été déterminées ou mesurées.

2. Appareil de traitement selon la revendication 1, dans lequel l'unité de correction (13) corrige au moins une première valeur de réserve de débit fractionnaire pour une position dans les artères coronaires (3) de l'être vivant en la remplaçant par une deuxième valeur de réserve de débit fractionnaire qui est mesurée à la même position dans les artères coronaires (3) de l'être vivant.

3. Appareil de traitement selon l'une quelconque des revendications précédentes, dans lequel l'unité de correction (13) ne corrige une première valeur de réserve de débit fractionnaire que lorsque la différence entre la première valeur de réserve de débit fractionnaire et la deuxième valeur de réserve de débit fractionnaire est supérieure à une valeur seuil prédéterminée.

4. Appareil de traitement selon l'une quelconque des revendications précédentes, dans lequel les premières valeurs de réserve de débit fractionnaire ont été calculées dans les 10 secondes suivant l'obtention des données d'imagerie non invasive des artères coronaires (3) de l'être vivant.

5. Appareil d'évaluation des données cardiaques d'un être vivant, comprenant
- un appareil de traitement (1) selon l'une quelconque des revendications 1 à 4; et
- une unité d'affichage (14) configurée pour afficher au moins une première valeur de réserve de débit fractionnaire et une deuxième valeur de réserve de débit fractionnaire correspondante pour une position correspondante dans les artères coronaires (3).

6. Appareil selon la revendication 5, comprenant en outre
- un dispositif d'imagerie (2) pour l'imagerie non invasive d'une région cardiaque d'un être vivant; le dispositif d'imagerie (2) comprenant une unité de traitement (21) pour déterminer les valeurs de réserve de débit fractionnaire; et
- un dispositif de mesure (31) pour la mesure dans l'artère des valeurs de réserve de débit fractionnaire.

7. Appareil selon la revendication 5, dans lequel le dispositif d'imagerie (2) comprend en outre une unité de traitement d'image (21) pour reconstruire des données d'image tridimensionnelles à partir de données de détection détectées par le dispositif d'imagerie (2).

8. Appareil selon l'une quelconque des revendications 5 à 7, dans lequel l'unité d'affichage (14) est configurée pour afficher des valeurs de réserve de débit fractionnaire corrigées chaque fois qu'une première valeur de réserve de débit fractionnaire est corrigée.
